# EUROPEAN PATENT SPECIFICATION

(11) **EP 0 909 274 B1**
(45) Date of publication and mention of the grant of the patent: **10.08.2005**
(21) Application number: 97906426.8
(22) Date of filing: 22.01.1997
(51) Int. Cl.: C07K 14/705

(54) **METHOD FOR PURIFICATION OF ERYTHROPOIETIN BINDING PROTEIN**
METHODE ZUR REINIGUNG EINES ERYTHRPOIETINBINDENDEN PROTEINS
PROCEDE DE PURIFICATION D'UN PROTEINE FIXANT L'ERYTHROPOIETINE

(30) Priority: 23.01.1996 US 10469 P; 18.07.1996 US 22129 P
(43) Date of publication of application: 21.04.1999
(73) Proprietor: Ortho-McNeil Pharmaceutical, Inc., New Brunswick, New Jersey 08933-7002 (US)
(72) Inventor: MIDDLETON, Steven, A., Flemington, NJ 08822 (US); JOHNSON, Dana, Upper Black Eddy, PA 18972 (US); MCMAHON, Frank, J., Whitehouse Station, NJ 08889 (US); MULKAHY, Linda, S., Yardley, PA 19067 (US); JOLLIFFE, Linda, K., Belle Mead, NJ 08502 (US)
(74) Representative: Mercer, Christopher Paul
(86) International application number: PCT/US1997/000932
(87) International publication number: WO 1997/027219

(56) References cited:
- NAGAO ET AL.: "Production and ligand-binding characteristics of the soluble form of murine erythropoietin receptor" BIOCHEMICAL AND BIOPHYSICAL RESEARCH COMMUNICATIONS, vol. 188, no. 2, 1992, pages 888-897,
- NAGAO ET AL: "Production and ligand-binding characteristics of the soluble form of murine erythropoietin receptor" BIOCHEMICAL AND BIOPHYSICAL RESEARCH COMMUNICATIONS, vol. 188, no. 2, 1992, pages 888-897,
- J.CELL.BIOL., vol. UPPL. 17, no. A, 1993, page 47,
- CLIN.RES., vol. 41, no. 2, 1993, page 134A,

## Description

### BACKGROUND OF THE INVENTION

The hematological process leading to the production and maturation of red blood cells is under the control of erythropoietin (EPO) (reviewed in Krantz, S.B. (1991) Erythropoietin. Blood 77, 419-434), a glycoprotein hormone primarily synthesized in the kidney. Commercially available human EPO is produced via recombinant DNA techniques and is known as recombinant human EPO (rhEPO). rhEPO has a molecular mass of approximately 36,000 Daltons, as determined by SDS-PAGE. The molecular mass of the protein backbone is 18,398 Daltons, which indicates that the entire molecule is heavily glycosylated. The carbohydrate residues are important for in vivo biologic activity.

In contrast to many other growth factors, the specificity of EPO for erythroid cells has lead to its development as a safe and efficacious therapeutic protein. The medical benefits of EPO have been well established in the treatment of anemia associated with chronic renal failure, cancer chemotherapy, and autologous predonation of blood. Due to the chronic nature of EPO therapy, it would be desirable to have an orally administered "second generation" molecule.

An understanding of the structural basis of the interaction of EPO with its receptor will aid in the design of new drugs, such as an oral anemia drug. Traditional methods of drug discovery are being supplemented by rational design approaches that attempt to make use of information about the structural basis of receptor-ligand interactions to develop molecular models. These models, in turn, are used to design molecules with therapeutic potential. The two approaches are complementary and rely on the ability to obtain information about the three-dimensional structure of the therapeutic target. The ability to produce EPO and its receptor and to assess the impact of Structural changes on protein function provides a means for testing hypothetical molecular models and contributes to the establishment of a structure activity relationship database for drug design.

The biological effect of EPO appears to be mediated, in part, through interaction with a cell membrane bound receptor which has previously been cloned (Jones, S.S., D'Andrea, A. D. , Haines, L.L., and Wong, G.G. (1990), Human erythropoietin receptor: Cloning, expression, and biological characterization, Blood 76, 31-35; Noguchi, C.T., Kyung, S.B., Chin, K., Wada, Y., Schecter, A.N. and Hankins, W.D. (1991) Cloning of the human erythropoietin receptor gene, Blood 78, 2548-2556; Maouche, L., Tournamile C., Hattab, C., Boffa, G., Carton J.-P. and Chretein, S. (1991) Cloning of the gene encoding the human erythropoietin receptor. Blood 78, 2557-2563). Currently there is considerable interest in the physical nature of the association cf EPO with the EPO receptor (EPOR) and an emerging technique for the analysis of this type of interaction is the generation of soluble receptors, also termed hormone binding proteins (Langer, J.A. (1990) Soluble ligand-binding fragments of polypeptide receptors in basic and applied research, Pharmaceutical Technology 14, 46-66). The process involves the engineering of suitable expression vectors encoding the extracellular domain of the receptor and the subsequent production and purification of the protein. Once obtained in active form, these soluble receptor fragments are useful in numerous assay formats and have improved utility in biophysical studies such as NMR or X-ray crystallography, since they can be employed in conditions free of the detergents required to solubilize membrane bound receptors. Some receptor fragments of this type, including IL-1 and IL-4, function to neutralize the biological effects of the hormone and appear to have therapeutic potential (Maliszewski, C.R. and Fanslow, W.C. (1990) Soluble receptors for IL-1 and IL-4: Biological activity and therapeutic potential, Trends in Biotech 8, 324-329).

Previous reports outline the development of systems for the production and purification of human and murine EPO binding protein (EBP) utilizing protein expression in eukaryotic cells and bacteria but with modest yields (Harris, K.W., Mitchell, R.A., and Winkleman, J.C. (1992) Ligand Binding Properties of the Human Erythropoietin Receptor Extracellular Domain Expressed in E. coli. J. Biol. Chem. 267, 15205-15209; Yet, M.-G and Jones, S.S. (1993) The Extracytoplasmic Domain of the Erythropoietin Receptor Forms a Monomeric Complex with Erythropoietin. Blood 82, 1713-1719; Nagao, M., Masuda, S., Abe, S., Ueda, M. and Sasaki, R. (1992) Production and ligand-binding characteristics of a soluble form of the murine erythropoietin receptor, Biochem. Biophys. Res. Comm. 188, 888-897).

The mechanism of EPO receptor activation has been suggested to reside in the dimerization of two EPO receptor molecules which results in subsequent steps of signal transduction [Watowich, S.S., Yohimura, A., Longmore, G.D., Hiltor., D.J., Yoshimura, and Lodish, H.F., Homodimerization and constitutive activation of the erythropoietin receptor. Proceedings of the National Academy of Sciences 89, 2140-2144 (1992)]. While the soluble EPO receptor [Johnson, D.L., Middleton, S.A., McMahon, F., Barbone, F., Kroon, D., Tsao, E., Lee, W.H., Mulcahy, L.S. and Jolliffe, L.K., Refolding, Purification and Characterization of Human Erythropoietin Binding Protein Produced in Escherichia coli, Protein Expression and Purification 7 104-113 (1996)] has advantages related to structure determination and ease of production, it likely does not represent a preformed template for receptor dimerization. In the search for peptides or small molecules which might bind to and activate the EPO receptor such a preformed dimerization template is a highly valuable root for the discovery, detection, and description of molecules with such activity. Use of receptor-Ig fusion molecules provide such templates and depending upon the assay format provide information on the ability of a given molecule or compound to detect non-productive as well as productive dimerization complexes.

### SUMMARY OF THE INVENTION

A new process for the production of highly pure human EPO binding protein (EBP) from fermentations of recombinant host cells is disclosed herein. The described process of the present invention produces a highly pure non-glycosylated EBP which retains the EPO receptor biological property of EPO (ligand) binding. The highly pure EBP is also useful in a wide variety of drug discovery techniques, including but not limited to, the design, discovery and production of ligand mimetics, the design, discovery and production of inhibitors of ligand binding, the design, discovery and production of agonists, antagonists and other modulators of ligand binding, and the production of crystal structures which allow the precise characterization of the EBP-ligand interactic site(s). The precise characterization of the EBP-ligand interaction site(s) is also useful for the the design, discover and production of ligand mimetics, the design, discovery and production of inhibitors of ligand binding, and the design, discovery and production of agonists, antagonists and other modulators of ligand binding.

### BRIEF DESCRIPTION OF THE DRAWINGS

Figure 1. The plasmid vector used for the expression of EBP in E. coli is shown.
Figure 2 Panels A and B. Reducing (Panel A) and non-reducing (Panel B) 10-20% SDS-PAGE of samples from EBP expression and purification studies are shown.
Figure 3, Panels A, B, C, and D: Demonstration of the detection of the "active" form of EBP is shown.
Figure 4, Panels A, B, C, and D. Demonstration of EBP purity and activity after purification by HIC and preparative size-exclusion chromatography is shown.
Figure 5, Panels A and B. Determination of EPO-EBP complex stoichiometry by C-4 reverse phase HPLC is shown.
Figure 6, Panels A and B. Equilibrium EPO binding to immobilized EBP and mass spectral analysis is shown.
Figure 7, Panels A and B. Competition of EBP for [¹²⁵I]EPO binding to cell associated native EPO receptors, is shown.

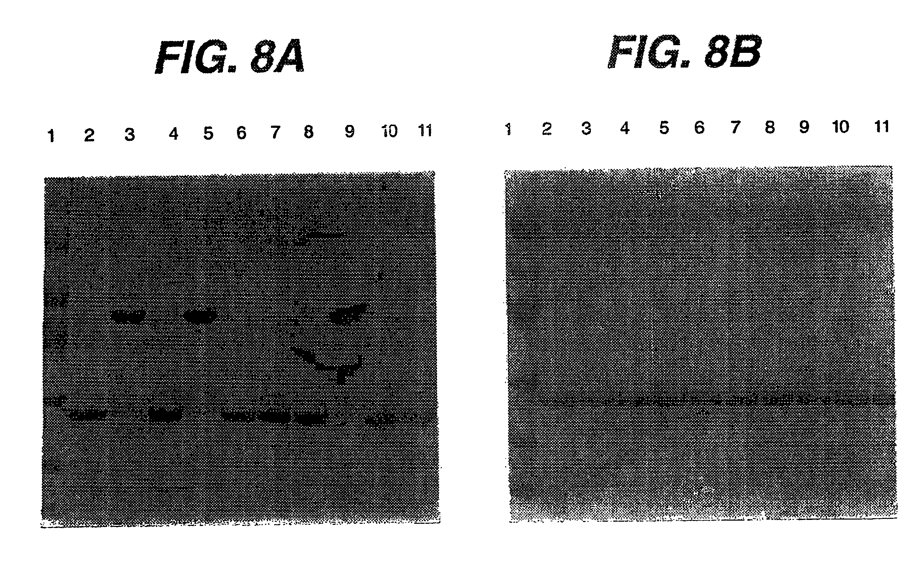

Figure 8, Panels A and B. Crosslinking of EBP proteins that were dimerized by different peptide ligands is shown by SDS-PAGE under nonreducing conditions (Panel A) and reducing conditions (Panel B).

### DETAILED DESCRIPTION OF THE INVENTION

The present invention is drawn to a process for the purification of erythropoietin binding protein (EBP) from microbial fermentations. The EBP purified by the process of the present invention is also useful as a therapeutic since it binds to erythropoietin, making the erythropoietin unavailable for interaction with the cell-associated receptor.

The term EBP as used herein refers to a form of the EPO receptor that lacks a substantial portion of the membrane anchorage domain and cytoplasmic domain, yet retains the ligand binding domain and the ability to bind erythropoietin. The term EBP-Ig as used herein refers to a recombinantly produced fusion protein comprising an immunoglobulin heavy chain including the hinge region, and EBP, a form of the EPO receptor that lacks a substantial portion of the membrane anchorage domain and cytoplasmic domain, yet retains the ligand binding domain and the ability to bind erythropoietin. The term EPO mimetic as used herein refers to a compound which binds to the EPO receptor and results in subsequent steps of EPO receptor-mediated signal transduction.

The process of the present invention is suitable for use with microbial fermentations in general. It is readily apparent to those skilled in the art that a wide variety of microbial cells are suitable for use in the process of the present invention, including but not limited to, fungal cells including yeast, and bacterial cells, including E. coli. A preferred microbial fermentation is a bacterial fermentation of cells containing the plasmid encoding the EBP cr an EBP fusion protein to be isolated and purified. A preferred bacterial fermentation is a fermentation of E. coli expressing the EBP or an EBP fusion protein to be isolated and purified. It is readily apparent to those skilled in the art that bacterial fermentations other than E. coli fermentations are suitable for use in the present invention. The microbial fermentation may be grown in any liquid medium which is suitable for growth of the bacteria being utilized.

The EBP to be isolated and purified by the process of the present invention can be expressed from any suitable EBP-encoding DNA sequence. The EBP-encoding DNA sequence may be derived from DNA encoding an EPO receptor which itself is derived from a cell line or cell type that expresses an EPO receptor.

The EBP-encoding DNA sequence may be contained on a variety of plasmids which can be high copy number per cell or low copy number per cell. The plasmids can also be of virtually any size. It is readily apparent to those skilled in the art that virtually any plasmid containing the EBP-encoding DNA sequence that results in the expression of the EBP in the recombinant host cells can be used in the process of the present invention.

The cloned EBP-encoding DNA obtained through the methods described herein may be recombinantly expressed by molecular cloning into an expression vector containing a suitable promoter and other appropriate transcription regulatory elements, and transferred into prokaryotic or eukaryotic host cells to produce recombinant protein. Techniques for such manipulations are fully described in Maniatis, T., Fritsch, E.F., Sambrook, J.; Molecular Cloning: A Laboratory Manual, Second Edition (Cold Spring Harbor Laboratory, Cold Spring Harbor, New York, 1989), and are well known in the art.

Expression vectors are defined herein as DNA sequences that are required for the transcription of cloned copies of genes and the translation of their mRNAs in an appropriate host. Such vectors can be used to express eukaryotic genes in a variety of hosts such as bacteria including E. coli, bluegreen algae, plant cells, insect cells, fungal cells including yeast cells, and animal cells.

Specifically designed vectors allow the shuttling of DNA between hosts such as bacteria-yeast or bacteria-animal cells or bacteria-fungal cells or bacteria-invertebrate cells. An appropriately constructed expression vector should contain: an origin of replication for autonomous replication in host cells, selectable markers, a limited number of useful restriction enzyme sites, a potential for high copy number, and active promoters. A promoter is defined as a DNA sequence that directs RNA polymerase to bind to DNA and initiate RNA synthesis. A strong promoter is one which causes mRNAs to be initiated at high frequency. Expression vectors may include, but are not limited to, cloning vectors, modified cloning vectors, specifically designed plasmids or viruses.

A variety of bacterial expression vectors may be used to express recombinant EBP in bacterial cells. Commercially available bacterial expression vectors which may be suitable for recombinant EBP expression include, but are not limited to pET vectors (Novagen), pRSET, pTrcHis, and pTrxFus vectors (Invitrogen), pQE vectors (Qiagen), pFLAG vectors (Eastman Kodak, International Biotechnologies Inc. pPRoEX™-1 (Life Technologies, Inc.), pKK vectors (Clonetech), pP_{L}-Lambda (Pharmacia), and pCal vectors (Stratagene).

A variety of fungal cell expression vectors may be used to express recombinant EBP in fungal cells such as yeast. Commerically available fungal cell expression vectors which may be suitable for recombinant EBP expression include but are not limited to pYES2 (Invitrogen). Pichia expression vectors (Invitrogen), and pYEUra3 (Clonetech).

A variety of insect cell expression vectors may be used to express recombinant EBP in insect cells. Commercially available insect cell expression vectors which may be suitable for recombinant expression of EBP include but are not limited to pBlueBacII (Invitrogen), pFastBacl (Life Technologies, Inc.), pBacPAK vectors (Clonetech), and pAc vectors (PHARMINGEN).

A variety of mammalian expression vectors may be used to express recombinant EBP in mammalian cells. Commercially available mammalian expression vectors which may be suitable for recombinant EBP expression, include but are not limited to, pMAMneo (Clontech), pcDNA3 (Invitrogen), pMClneo (Stratagene), pXT1 (Stratagene., pSG5 (Stratagene), EBO-pSV2-neo (ATCC 37593) pBPV-1(8-2) (ATCC 37110), pdBPV-MMTneo(342-12) (ATCC 37224), pRSVgpt (ATCC 37199), pRSVneo (ATDC 37198), pSV2-dhfr (ATCC 37146), pUCTag (ATCC 37460), lZD35 (ATCC 37565), and pEe12 (Celltech). Cell lines derived from mammalian species which may be suitable for expression and which are commercially available, include but are not limited to, CV-1 (ATCC CCL 70), COS-1 (ATCC CRL 1650), COS-7 (ATCC CRL 1651), CHO-K1 (ATCC CCL 61), 3T3 (ATCC CCL 92), NIH/3T3 (ATCC CRL 1658), HeLa (ATCC CCL 2), C127I (ATCC CRL 1616), BS-C-1 (ATCC CCL 26), MRC-5 (ATCC CCL 171), L-cells, HEK-293 (ATCC CRL1573), and NS0 (ECACC85110503).

DNA encoding EBP may be cloned into an expression vector for expression in a recombinant host cell. Recombinant host cells may be prokaryotic or eukaryotic, including but not limited to bacteria such as E. coli, fungal cells such as yeast, insect cells including but not limited to drosophila and silkworm derived cell lines, and mammalian cells and cell lines.

The expression vector may be introduced into host cells via any one of a number of techniques including but not limited to transformation, transfection, protoplast fusion, lipofection, and electroporation. The expression vector-containing cells are clonally propagated and individually analyzed to determine whether they produce EBP protein. Identification of EBP expressing host cell clones may be done by several means, including but not limited to immunological reactivity with anti-EBP, and the presence of host cell-associated EBP activity. Likewise, identification of EBP fusion protein expressing host cell clones may be done by several means, including but not limited to immunological reactivity with anti-EBP antibodies, and the presence of host cell-associated EBP activity, as well as immunological reactivity with antibodies specific for the nonEBP portion of the fusion protein, and other host cell-associated activities of the nonEBP portion of the fusion protein.

Because the genetic code is degenerate, more than one codon may be used to encode a particular amino acid, and therefore, the amino acid sequence can be encoded by any of a set of similar DNA oligonucleotides. Only one member of the set will be identical to the EBP sequence but will be capable of hybridizing to EBP DNA even in the presence of DNA oligonucleotides with mismatches under appropriate conditions. Under alternate conditions, the mismatched DNA oligonucleotides may still hybridize to the EBP DNA to permit identification and isolation of EBP-encoding DNA.

It is known that there is a substantial amount of redundancy in the various codons which code for specific amino acids. Therefore, the processor this invention is also directed to those EBP-encoding DNA sequences which contain alternative codons which code for the eventual translation of the identical amino acid. For purposes of this specification, a sequence bearing one or more replaced codons will be defined as a degenerate variation. Also included are mutations either in the DNA sequence or the translated protein which do not substantially alter the ultimate physical properties of the expressed protein. For example, substitution of valine for leucine, arginine for lysine, or asparagine for glutamine may not cause a change in functionality of the polypeptide. In addition, the alteration of naturally occurring glycosylation sites by site directed mutagenesis using techniques well known in the art may result in the production of a protein which is no longer glycosylated at a particular site. It is readily apparent to those skilled in the art that an EBP modified in such a manner is an EBP variant that can be purified by the process of this invention.

It is known that DNA sequences coding for a peptide may be altered so as to code for a peptide having properties that are different than those of the naturally-occurring peptide. Methods of altering the DNA sequences include, but are not limited to site directed mutagenesis. Examples of altered properties include but are not limited to changes in the affinity of an enzyme for a substrate or a receptor for a ligand.

As used herein, a "functional derivative" of EBP is a protein that possesses a biolcaical activity (either functional or structural) that is substantially similar to the EBP biological activity of EPO binding. The term "functional derivatives" is intended to include the "fragments," "variants," "degenerate variants," "analogs" and "homologues" or to "chemical derivatives" cf EBP. The term "fragment" is meant to refer to any polypeptide subset of EBP. The term "variant" is meant to refer to a molecule substantially similar in structure and function to either the entire EBP molecule or to a fragment thereof. A molecule is "substantially similar" to EBP if both molecules have substantially similar structures or if both molecules possess similar biological activity. Therefore, if the two molecules possess substantially similar activity, they are considered to be variants even if the structure of one of the molecules is not found in the other or even if the two amino acid sequences are not identical. The term "analog" refers to a moiecule substantially similar in function to either the entire EBP molecule or to a fragment thereof.

EBP purified by the process of the present invention is useful in a wide variety of drug discovery techniques, including but not limited to, the design, discovery and production of ligand mimetics, the design, discovery and production of inhibitors of ligand binding, the design, discovery and production of agonists, antagonists and other modulators of ligand binding, and the production of crystal structures which allow the precise characterization of the EBP-ligand interaction site(s). Compounds which are EPO mimetics may be DNA, RNA, peptides, proteins, or non-proteinaceous organic molecules.

Pharmaceutically useful compositions containing EBP, may be formulated according to known methods such as by the admixture of a pharmaceutically acceptable carrier. Examples of such carriers and methods of formulation may be found in Remington's Pharmaceutical Sciences. To form a pharmaceutically acceptable composition suitable for effective administration, such compositions will contain an effective amount of the peptide or protein, DNA, RNA, or modulator.

Following expression of EBP in a recombinant host cell, EBP may be recovered to provide EBP in active form. Microbial cells containing the EBP expression plasmid are harvested from the fermentation medium to provide a cell paste, or slurry. Any conventional means to harvest cells from a liquid medium is suitable, including, but not limited to centrifugation or microfiltration.

The harvested cells are lysed in a suitable lysis buffer. The insoluble proteins are separated from the soluble proteins by conventional methods known in the art, such as centrifugation and microfiltration. The EBP may be present in either or both of the soluble and insoluble protein fractions of the cell lysate.

The EBP produced in the system described below accumulates as an insoluble protein which is then recovered and refolded to obtain an active form of the protein. It is readily apparent to those skilled in the art that a wide variety of expression vectors and host cells can be used in the present method and that the EBP to be expressed may be engineered to contain or lack a signal sequence. In addition, the purification process of the present invention can be used to purify EBP from a mixture of expressed EBP's comprising EBP with and without a signal sequence.

The insoluble protein fraction is collected and solubilized in a suitable solubilization buffer. The solubilized proteins are again separated from the insoluble proteins as described above, and the soluble EBP protein fraction is collected. The solubilized EBP is refolded by incubation for a suitable period of time in a suitable refolding buffer, by conventional methods known in the art. The refolded EBP is then subjected to hydrophobic interaction chromatography using a suitable hydrophobic interaction chromatographic matrix. Examples of suitable hydrophobic interaction chromatographic matrices include, but are not limited to, alkyl or aromatic substituted silica or polymer based resins such as Octyl-Sepharose, Butyl-Sepharose, Phenyl-Sepharose (Pharmacia); Ethyl-, Propyl-, Butyl, Pentyl-, Hexyl-, Octyl, Decyl-, Dodecyl-, Phenylbutylamine-or Phenyl substituted beaded agarose (Supleco); or Phenyl or Butyl Toyopearl with Phenyl-Toyopearl 650 M being preferred. The proteins are eluted from the hydrophobic interaction column using a suitable elution buffer. The fractions containing active EBP as determined by the methods described herein, are collected. The active EBP is found primarily in the first major protein peak eluted from the column. The column fractions containing the active EBP are pooled and subjected tc high-performance size exclusion chromatography (HP-SEC) using a suitable HP-SEC matrix. Suitable HP-SEC matrices include, but are not limited to, porous silica and polymeric gel filtration media including Sephacryl, Sepharose, Superdex and Sephadex Sized Resins; Toyopearl HW, Progel TSK (Supelco) with Bio-Sil SEC-250 (BioRad) or TosoHaas G3000SW being preferred. The fractions from the HP-SEC column that contain EBP activity are pooled and represent extremely pure EBP which binds EPO. This extremely pure EBP is suitable for the uses described herein. It is readily apparent to those skilled in the art that varying the level of EBP purity will provide EBP that may be suitable for the uses disclosed herein.

The initial recovery of EBP from the refold mix may also be accomplished using anion exchange chromatography. Application of a gradient of increasing salt concentration in a suitable buffer results in the elution a two major absorbance peaks of protein with the first major peak containing active EBP. Suitable anion exchange media include, but are not limited to, substituted polymeric or non-porous resins with DEAE or QAE functional modifications including TSK-5w-DEAE, DEAE Sepharose, QAE Sepharose or DEAE Sepharose with DEAE Sepharose FastFlow being preferred. Active EBP recovered in this fashion can be further purified by high-performance size exclusion chromatography (HP-SEC).

The refolding of EBP produced in E. coli using the process described herein has identified a long lived but transient protein folding intermediate which ultimately relaxes into the active form of the protein. Active protein purified from 24 hr refolding mixtures appeared identical to that refolded for seven days in ligand binding determinations supporting the conclusion that only a single active form of the protein, independent of refolding duration, was purified by these methods. HP-SEC studies have demonstrated that the intermediate appears to have a greater hydrodynamic volume than the active form of the protein with a retention time corresponding to a protein with a mass approximately twice that of the active form of the protein. This may indicate the participation of a dimeric folding intermediate or an intermediate in which only one of the two predicted domains of the protein is folded resulting in an increased hydrodynamic volume.

The soluble ligand binding domain described herein neutralizes the proliferative properties of EPO as detected by the dose dependent neutralization of an EPO responsive cell line. A molecule with the ability to neutralize EPC, such as the EBP purified by the process of the present invention, may have utility in the treatment of EPO responsive proliferative disorders such as erythroleukemia and polycythemia. EBP can also be used to neutralize EPO in vivo.

The process of the present invention allows for the production of active, unglycosylated EBP potentially suitable for a variety of uses, including but not limited to, drug design and discovery, as a therapeutic, and for structural investigations by NMR and crystallography. Production of the protein in bacteria permits the facile incorporation of the stable carbon and nitrogen isotopes required for heteronuclear NMR studies and limits the potentially complicating factor of heterogeneity induced by the glycosylation of the protein if produced by mammalian cell culture. In particular, the process for EBP purification of the present invention produces a protein that is suitably pure for crystal propagation, thereby enabling crystallography studies.

In addition, recombinant EBP can be separated from other cellular proteins by use of an immunoaffinity column made with monoclonal or polyclonal antibodies specific for EBP.

The following examples are provided to illustrate the present invention.

### Example 1

### Construction of the EBP Bacterial Expression Vector.

To direct expression to the periplasmic space, the pelB signal sequence (Lei, S-P., Lin, H-C., Wang, S. S., Callaway, J., and Wilcox, G. (1987), Characterization of the Erwina carotovora pelB gene and its product pectate lyase, J. Bact.

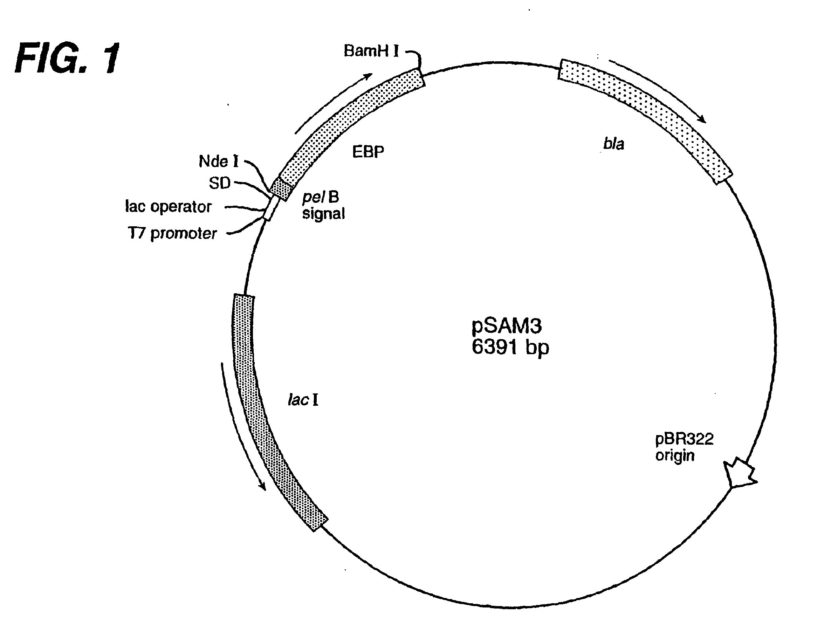

164, 4379-12; Studier, F.W., Rosenberg, A.H., Dunn, J.J., & Dubendorff, J.W. (1990) Use of T7 RNA polymerase to direct expression of cloned genes, Methods Enzymol. 185: 60-89) was fused to the N-terminus of the mature hEPOR (Jones, S.S., D'Andrea, A.D., Haines, L.L., and Wong, G.G. (1990). Human erythropoietin receptor: Cloning, expression, and biological characterization. Blood 76, 31-35) by overlap extension PCR (Horton, R.M., Hunt, H.D., Ho, S.N., Pullen, J.K., and Pease, L.R. (1989) Engineering hybrid genes without the use of restriction enzymes: gene splicing by overlap extension, Gene 77, 61-68). Two separate DNA fragments, one encoding the pelB signal sequence and one encoding amino acids 1-225 of the extracellular domain of the mature hEPOR were generated by PCR. The terminal 16 nucleotides of the pelB PCR fragment were designed to be identical to the first 16 nucleotides of the EBP PCR fragment so that the two fragments could be used as templates for the overlap extension PCR. The 5' and 3' primers used for the overlap extension PCR were designed to introduce Nde I and BamH I restriction sites, respectively, for subsequent cloning into the bacterial expression plasmid. In addition, the 3' primer introduced a stop codon in place of amino acid 226 of the mature hEPOR. The pelB-EBP PCR fragment was ligated into the Nde I and BamH I sites of the T7 promoter expression vector pET11a (Novagen, Inc., Madison, WI) and its sequence confirmed by DNA sequencing. For bacterial expression, the resultant construct, called pSAM3 (Figure 1), was transformed into E. coli strain, BL21(DE3)pLysS, carrying the T7 RNA polymerase on the chromosome under control of the IPTG inducible lac promoter (Studier, F.W., Rosenberg, A.H., Dunn, J.J., & Dubendorff, J.W. (1990) Use of T7 RNA polymerase to direct expression of cloned genes, Methods Enzymol. 185: 60-89). EBP is expressed from the T7 promoter under regulation of the lac operator. The genes encoding the lac repressor (lacI) and ampicillin resistance (bla) are also present on the plasmid. Typically, cultures were grown to an OD₆₀₀ of 0.6 to 0.9 in M9ZB medium (Studier, F.W. et al., (1990) supra) containing 100 pg/mL ampicillin and 25 µg/mL chloramphenicol at which time protein expression was induced with a final concentration of 1 mM IPTG. After a further 2 to 3 hours incubation, cells were harvested and the EBP was isolated and purified as described herein.

### Fermentation.

Parental E. coli strain BL21(DE3)pLysS (Studier, F.W. et al., (1990) supra) transformed with plasmid pSAM3 (designated SM4) was maintained in M9ZB amp/cap medium [M9ZB amp/cap media per liter: 10 g N-Z-Amine A, 100 ml 10X M9 Minimal Salts (Sigma Chemical Co. St. Louis, MO.), 5 g NaCl, 1 mM MgSO₄, 2% D-glucose, 100 ma ampicillin and 25 mg chloramphenicol] as 8% glycerol stock solutions of an OD₆₀₀=1.0 culture. High density fermentation was carried out in a CF-3000 Fermenter (Chemap Inc., South Plainfield, NJ) fitted with a 10 liter stirred liter tank. Enriched M9ZB media containing (per liter) 20 g N-Z-amine A, 5 g NaCl, 10 g M9 Minimal Salts, 0.12 g MgSO₄, 0.2 g glucose, 100 mg ampicillin and 25 mg chloramphenicol was prepared, filtered through a 0.22 µm Milli-Pak 40 (Millipore Corp.) device and pumped into the fermenter. After equilibration of the medium at 37°C and 20% dissolved oxygen at pH 6.8 the fermenter was inoculated with 50 ml of the transformed E. coli glycerol stock (see above). Prior to induction, the pH was maintained via glucose feed (400 g glucose to 1 liter of enriched M9ZB media). When an OD₆₀₀ value of 25.0 was obtained, protein overexpression was induced by the addition of IPTG to a final concentration of 1 mM and the pH reduced to 6.1 by the addition of HCl. The induction phase was allowed to proceed overnight. At harvest the OD₆₀₀ value was 36 and 445 g of cell paste was recovered by centrifugation. The final pH of 6.1 greatly slows the doubling time and the final pH required to limit the rate of cell growth is dependent upon reliable pH control and probe calibration.

The constructed expression vector encodes the initial 225 N-terminal residues of the mature human erythropoietin receptor and includes a pelB signal sequence which, it was anticipated, would direct the expressed protein to the periplasmic space of E. coli to yield soluble, properly folded EBP. Western blot analysis of soluble periplasmic protein preparations and growth media showed low levels of protein present in these preparations after induction. Boiled extracts of cultures analyzed by SDS-PAGE demonstrated two intense protein bands present in induced cultures which were absent in cultures grown under identical conditions except for induction with IPTG. The apparent molecular weight of the smaller species was estimated at 27 kDa while the slower migrating species appeared to be approximately 1.5 kDa larger. Recovery of insoluble protein at the analytical scale led to preparations of protein which appeared to be mainly these two species. N-terminal sequence analysis of this protein, after solubilization, refolding and buffer exchange to PBS, indicated two protein sequences; one which corresponded to the first six amino acids expected for the mature N-terminal sequence of the EPO receptor, and a second that constitutes the first six amino acids of the N-terminal pelB signal sequence. These data, taken together, show that the expression system leads to production of two protein forms, both insoluble and presumably located in inclusion bodies, one which corresponds to EBP having a properly processed amino terminus and one that retains the encoded pelB signal sequence. A signal sequence appears to be required for production of the EBP in this expression system, since a version of plasmid pSAM3 lacking the pelB signal sequence did not express EBP in E. coli strain BL21(DE3)pLysS. Other plasmid constructs or other E. coli strains may be constructed which do not require the presence of a signal sequence for high level production of the EBP. An alternative construct in which the ompT signal sequence was employed has also been observed to generate considerable amounts of overexpressed insoluble EBP.

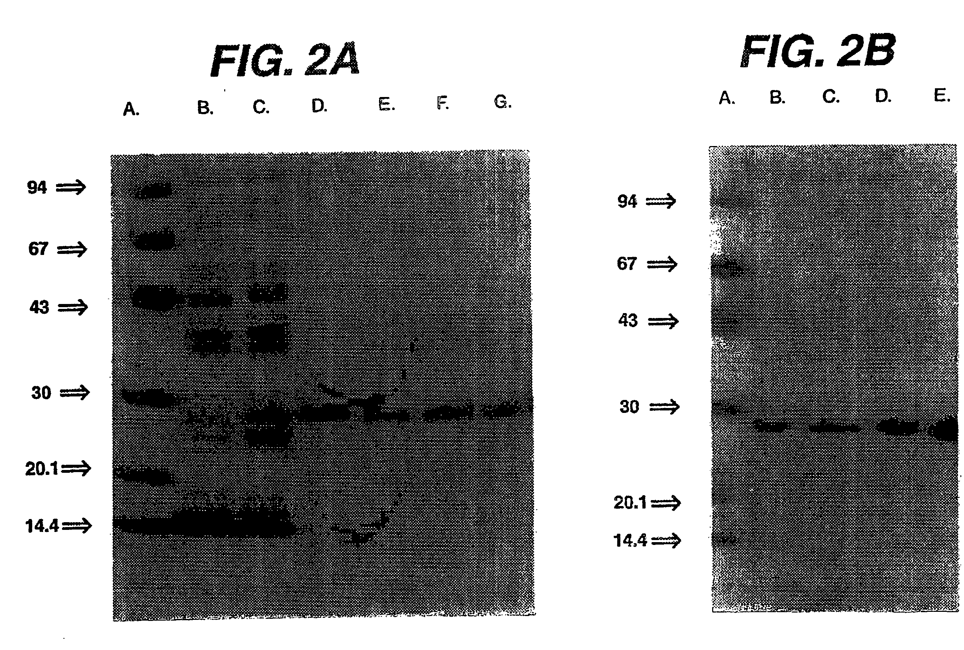

Additional fermentations were performed which varied the distribution of the signal sequence "on" and signal sequence "off" forms of EBP. Initial controlled fermentation studies revealed induced cultures with a greater amount of processed EBP that had a lower final pH. Subsequently an experimental protocol for the production of completely processed EBP was developed. The final fermentation process features a carefully controlled oxygen level and a regulated reduction in the pH of the culture upon induction of protein expression. This results in the expression of a protein species which has an apparent molecular weight of about 27 kDa upon SDS-PAGE analysis (Figure 2, panel A, lane B versus lane C). Panel A (reduced) samples were as follows: A. Pharmacia LMWM; B. Before induction; C. After induction; D. Refold mix; E. HIC pool; F. HP-SEC pool (2.5 µg); G. HP-SEC pool (5.0 µg). Panel B (non-reduced) samples were as follows: A. Pharmacia LMWM; B. Refold mix; C. HIC pool; D. HP-SEC pool (2.5 µg); E. HP-SEC pool (5.0 µg).

Fermentation under less rigorous control may also be used to obtain EBP which appears as a mixture of "signal sequence on" and "signal sequence processed" material as described above. Insoluble EBP obtained from these fermentations may also be refolded and purified by the methods described herein to yield active protein with a properly processed amino terminus. The refolding and purification result in the separation of the protein form which retains the unprocessed signal sequence.

### Example 2

### Protein Recovery and Protein Refolding.

Given the high level expression of insoluble protein we sought conditions for the recovery and refolding of the inclusion bodies to obtain active protein. Conditions were developed based on a number of factors to be considered in the refolding of recombinant proteins (Marston, F.A.O. (1986) The purification of eukaryotic polypeptides synthesized in Escherichia coli. Biochemical Journal 240, 1-12; Light, A. (1985) Protein solubility, protein modifications and protein folding, BioTechniques 3, 298-306; Schein, C.H. (1990) Solubility as a function of protein structure and solvent components, Bio/Technology 8, 308-317). Centrifugation proved adequate for recovery of the inclusion bodies which were found to be optimally solubilized at a concentration of 3.5 M urea. This concentration of urea effectively solubilized >90% of the EBP but left several contaminating proteins in the insoluble fraction. Early experiments on refolding conditions suggested that the process could be followed by high performance-size exclusion chromatography (HP-SEC). The use of HP-SEC was based on the notion that if the pathway of protein refolding proceeds toward either correctly folded active product or various types of protein aggregates, then the use of an analytical technique which discriminates on the basis of molecular volume should predict the efficiency of a refolding protocol alone. Therefore, the protein refolding process was monitored by high performance size exclusion chromatography (HP-SEC) on a Waters 625 HPLC system equipped with a Waters 991 detector. Separations were performed at ambient temperature on a BioSil SEC-250 (7.8 x 300 mm) (BioRad) column or a G-3000 SWXL (7.8 x 300 mm) column (Supelco, Bellfonte, PA) which provide comparable resolution. The analytical column was equilibrated in 10 mM sodium phosphate, pH 7.2, 150 mM NaCl, at a flow rate of 1 ml/min and was monitored at 220 nm. The active form of the protein was detected by peak shift analysis through the addition of 10 µg of purified recombinant human EPO (1.7 mg/ml, specific activity 120 units/pg) to a 100 µl aliquot of the bulk refold solution followed by HP-SEC analysis. This chromatogram was then compared to the chromatographic resolution of a 100 ul aliquot of bulk refold solution mix performed in the absence of EPO.

Part of the cell paste (74.3 g) from the above controlled fermentation was lysed in 0.3 1 of EBP lysis buffer (10 mM TRIS-ECl, pH 7.5, 150 mM NaCl) containing 51 mg of phenylmethylsulfonyl fluoride, 600 mg egg white lysozyme (Calbiochem), 1 mM MgCl₂, and 12,500 units of Benzonase (EM Science) and in total termed EBP Lysis Buffer. This mixture was incubated at room temperature for 1.5 hr with occasional agitation. The resulting lysate was centrifuged at 12,000 x g for 15 min at 4°C. The supernatant was discarded and pellet was dispersed by application of a one minute burst of sonication after the addition of 0.3 1 of TE buffer (TE buffer: 10 mM TRIS-HCl, pH 7.5, 1 mM EDTA, 3% NP-40 [protein grade, Calbiochem]). The resulting suspension was centrifuged for 8000 x g for 15 min at 4°C. The supernatant was discarded and the insoluble proteins washed with 0.3 1 of water. The pellet was resuspended by sonication (20 sec). Insoluble protein was recovered by centrifugation (12,000 x g, 15 min, 4°C). The wet weight of protein recovered was about 3.5 g after decanting the wash supernatant.

The insoluble protein pellet was resuspended in 9 ml of solubilization buffer per gram of protein (solubilization buffer: 0.1 M TRIS-HCl, pH 8.5, 3.5 M urea, 10 mM lysine, 10 mM dithiothreitol) and was centrifuged at 12,000 x g for 15 min at 4°C. Lysine was added to act as a scavenger of amine reactive cyanates possibly present in the urea (Marston, F.A.O. and Hartley, D.L. (1990) Solubilization of protein aggregates, Methods Enzymol. 182, 264-276). At this point, the recovered supernatant can be incubated at 4°C overnight as a convenient stopping point or carried on to the refolding step. The OD₂₈₀ of the solubilized protein was determined against solubilization buffer (21.7 A.U./ml) and 30 ml of the solubilized protein was diluted to 1020 ml in refold buffer (refold buffer: 0.05 M TRIS-HCl, pH 8.5, 3 mM EDTA, 10 mM lysine, 2 mM reduced glutathione, 0.2 mM oxidized glutathione) and stored at 4°C. Final OD₂₈₀ of the refold mix was 0.64.

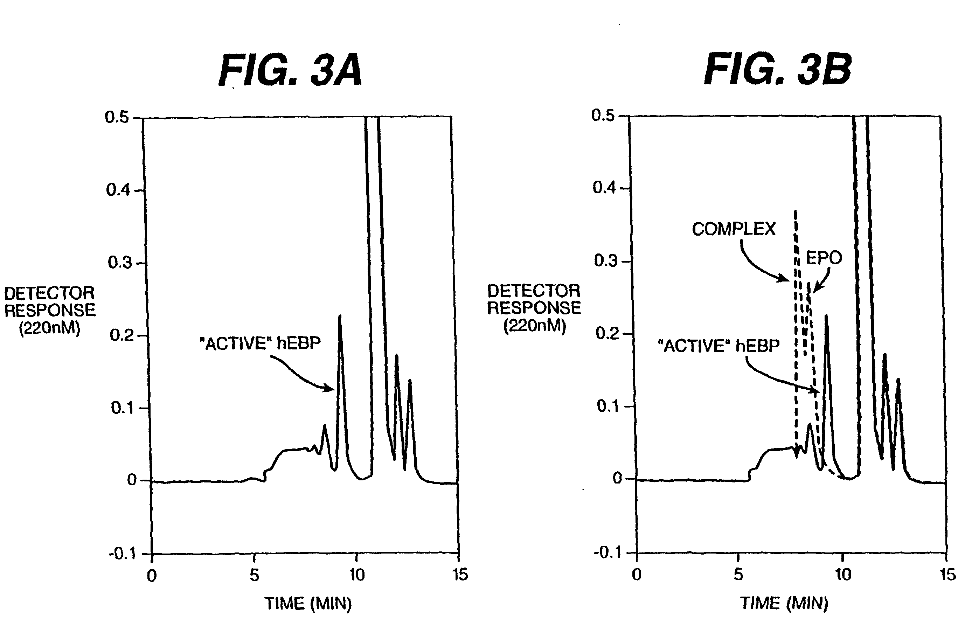

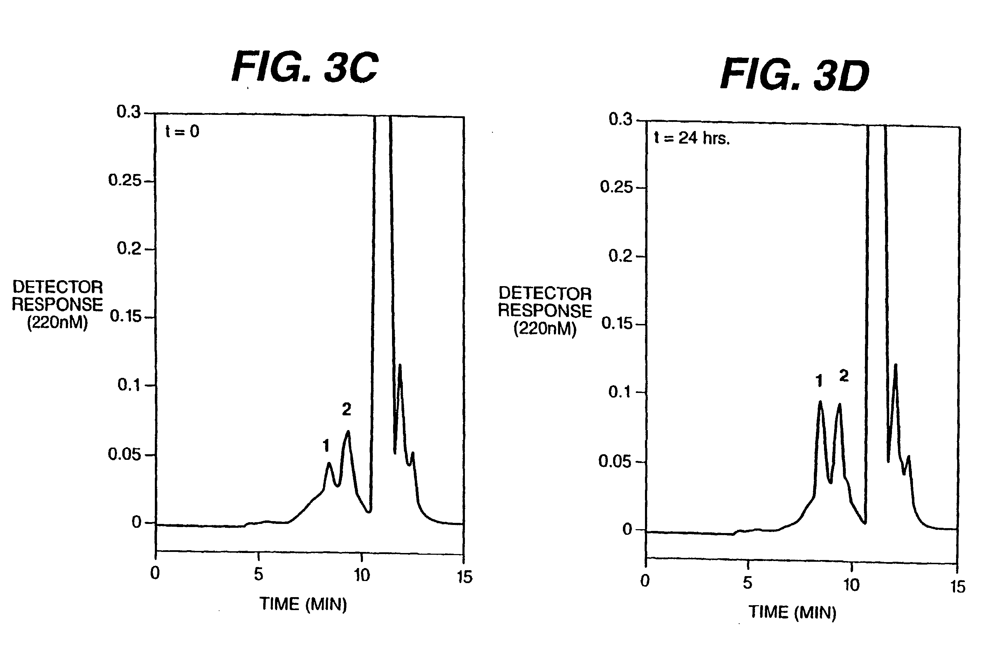

A qualitative estimation of ligand binding activity was possible by performing two HP-SEC experiments with the refold mixture, one with added ligand and one without (Figure 3). Insoluble EBP was solubilized as described herein and incubated at 4°C for six days (Figure 3, Panels A and B). As shown in Figure 3, Panel A, when the refold mix alone was injected onto an analytical SEC column an absorbance peak was observed at approximately 9.5 min which corresponds to a standard calibrated molecular weight of about 25,000 Daltons. This value is in good agreement with the predicted 24,724 molecular mass of EBP. Upon the addition of 8.5 µg of EPO to the mixture and subsequent chromatographic resolution, an EPO-EBP complex peak was detected at about 8.1 min and an EPO peak at about 8.5 min (Figure 3, Panel E, overlaid onto the experiment shown in Figure 3, Panel A). The absorbance peak at about 9.5 min is almost completely depleted demonstrating the presence of an EBP form which can interact with EPO to create a species with a greater apparent solution phase molecular mass. Only the 9.5 min peak appears to interact with EPO indicating a single active species. The peaks eluting after 10 min have calibrated molecular masses of less than 3000 Daltons. Figure 3, Panel C, shows a significant amount of properly refolded protein is observed immediately after dilution of the 3.5 M urea denaturant solution to 0.1 M urea as evidenced by the peak at the 9.5 min elution time (peak 2). After 24 hrs incubation at 4°C, an increase is observed in both the 9.5 min (Peak 2) active protein form and a species eluting at 8.2 min (Peak 1) as shown in Figure 3, Panel D. The appearance of these forms appears to be at the expense of higher molecular weight protein forms with retention times of 6-8 min and from insoluble protein in the refolding mix. As shown in Figure 3, Panel A the 8.2 min protein form gradually decays and the absorbance appears in the active protein peak. These experiments produce clear shifts of a protein of Mᵣ= 25,000 upon the addition of EPO to an aliquot of the refolding mix (Figure 3, Panel A and B). Only the Mᵣ= 25,000 peak appears to shift upon the addition of EPO indicating the presence of a single active species under these analysis conditions.

The HP-SEC study of the refolding mix over time resulted in the observation that a species of Mᵣ= 52,000 slowly converted into the active Mᵣ= 25,000 species. (Figure 3, Panel C, peaks 1 and 2 compared with Figure 3, Panel A [after 6 days of refolding time]). The refold solution remained slightly cloudy and it was presumed that the insoluble protein giving rise to the opaque solution was slowly entering solution giving rise to additional active and inactive protein species over the initial 24 hours of refolding (Figure 3, Panels C and D). This metastable species was observed to be long lived and appeared not to be related to a disulfide bonded species, since over the course of the lengthy refolding process non-reducing SDS-PAGE experiments demonstrated no differences with time. This study suggested that continued incubation at 4°C resulted in the accumulation of additional active product at the expense of the inactive material designated peak #1 (Figure 3, Panel A) and thus the refolding process was typically carried out for 5-7 days (Figure 3, Panel C vs. Panel A). Protein with an analytical HP-SEC elution time of 8.2 min (Figure 3, peak 1) was isolated by preparative HP-SEC and analyzed by SDS-PAGE. Under both reducing and non-reducing conditions the protein appeared to be monomeric. Since this peak ultimately decreases relative to the 9.5 min peak (Figure 3, peak 2) after several days refolding time (Figure 3, Panel A) it is suggested that this species might represent a refolding intermediate.

### Example 3

### Protein Purification.

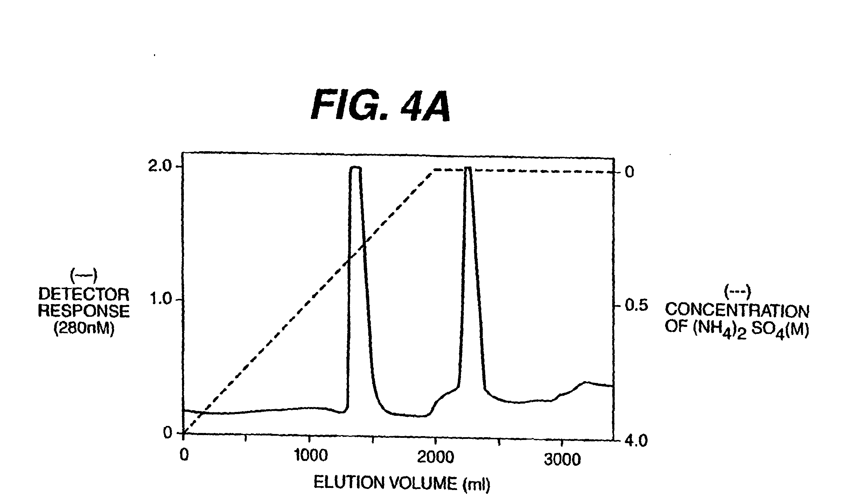

After six days refolding time, the final refold mix was warmed to 37°C and adjusted to 1 M (NH₄)₂SO₄ by the addition of 3.5 M (NH₄)₂SO₄ in 20 mM TRIS-HCl, pH 7.5 and additional 20 mM TRIS-HCl, pH 7.5. The final adjusted volume of the 1 M (NH₄)₂SO₄ refold mix was 1.5 liter. Analytical HP-SEC analysis of the solution revealed that, in part, the higher molecular weight forms in the refold mix were precipitated from solution. After filtering through a 0.45 µm filter, this solution was applied to a column (5 cm x 16 cm) of Phenyl-Toyopearl TSK 650 M (Supelco) at a flow rate of 8 ml/min at room temperature. The column was previously equilibrated with 1 M (NH₄)₂SO₄/ 20 mM TRIS-HCl, pH 7.5 (Buffer A). Upon completion of the column loading, the absorbance (280 nm) cf the column effluent was reduced to base line by washing with Buffer A. The protein bound to the column was eluted with a 2000 ml linear gradient of 20 mM TRIS-HCl, pH 7.5 (Buffer B), starting from 100% Buffer A, followed by 1000 ml of Buffer B. A flow rate of 8 ml/min was maintained over the course of the experiment. Fractions of 2 min duration were collected. Two major peaks of absorbance were eluted: one at near 80% Buffer B and another after approximately 200 ml 100% Buffer B Figure 4A. Analytical HP-SEC of the column fractions (100 µl) indicated that the first absorbance peak contained the active protein and fractions 85-102 were pooled and concentrated by ultrafiltration (Amicon YM-10 membrane). The recovered material was filtered through a 0.45 um filter before the next chromatography step.

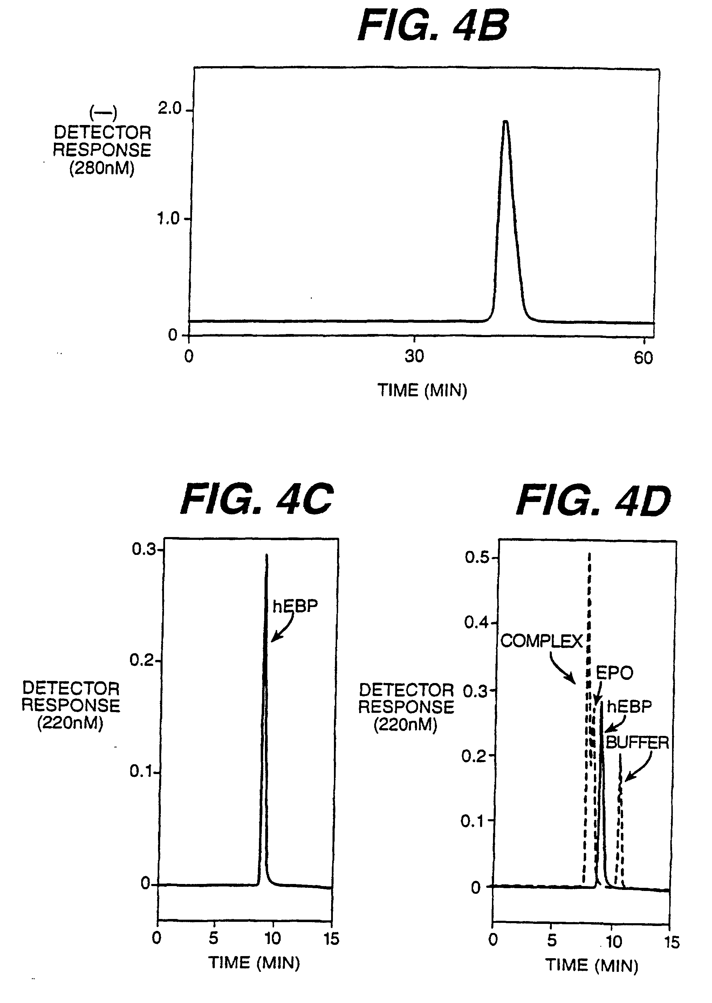

The recovered pool (22 ml, 60 mg protein) was subjected to HP-SEC on a Bio-Sil SEC-250 column (21.5 x 600 mm, BioRad) equipped with a Bio-Sil precolumn (7.5 x 21.5 mm) equilibrated with PBS, pH 7.5 at 4 ml/min. Injections of 4.5 ml were made and the entire pool was resolved in five chromatographic experiments. One minute fractions were collected and the active fractions from each run (fractions 40-42) were pooled and concentrated by ultrafiltration in Centriprep 10 devices (Amicon) [Figure 4B]. The recovered pool (23 ml, 2.04 mg/ml, 46 mg) was analyzed for activity by HF-SEC EPO binding analysis (see below) and for purity by non-reducing and reducing SDS-PAGE analysis (see figure 2). SDS-PAGE geis (10-20% gradient SDS-PAG plates, 84 x 70 x 1.0 mm, Integrated Separation Systems, Natick, MA) were stained with Coomasie Brilliant Blue R-250.

Upon analytical HP-SEC this protein appears as a single species with an elution time of 9.5 min with no trace of high molecular weight aggregates (Figure 4, Panel C). When mixed with an excess of EPO prior to chromatographic resolution, the EBP observed at 9.5 min is converted to a complex with a retention time of approximately 8.2 min (Figure 4, Panel D overlaid with experiment shown in Figure 4, Panel C). If EPO was added at a level below excess one observes the complete depletion of the EPO peak at 8.5 min and the complex peak at 8.2 min remains. A final EBP recovery yield of approximately 0.6 mg active protein per gram of wet cell weight has been observed with 33% overall yield from the refolding stage to apparently homogeneous protein. Table 1 shows the result of the purification of refolded EBP from E. coli.

**Table 1**

| Fraction | Total Protein (mg) | Active EBP¹ (mg) | Yield (%) |
|---|---|---|---|
| Refold mix | 195 | 138 | 100 |
| HIC | 60 | 58 | 42 |
| SEC | 46 | 46 | 33 |

| | | | |
|---|---|---|---|
| Note: Starting from 74 .3 grams of cells (wet weight). 1 - calculated from SEC peak area, based on % of active protein | | | |

### EXAMPLE 4

### Preparation of EPO-EBP Complex and Stoichiometry Determination.

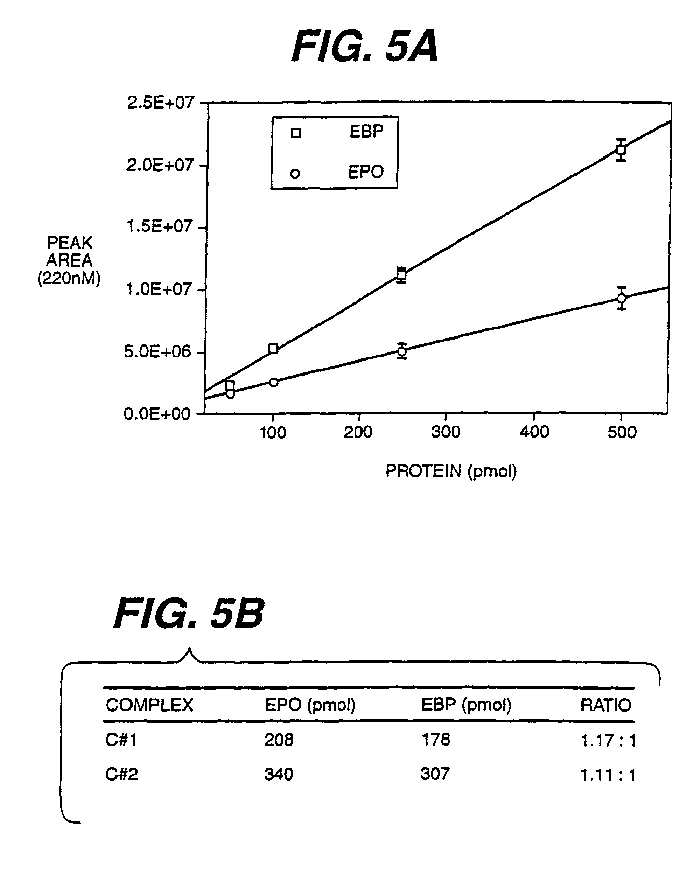

The stoichiometry of EBP and EPO in the binding complex was further examined by the isolation of EPO-EBP complex by HP-SEC, and the estimation of the ratio of protein within this mixture by C-4 reverse phase chromatography as described below. A standard curve of detector response for EPO and EBP (Figure 5, Panel A) was generated which demonstrated a linear detector response with a low level of repetitive error (1.0 S.D.) over three different analytical runs at each amount of analyte as indicated by the error bars. Figure 5, Panel B contains the results of the analysis of two different lots of EPO-EBP complex purified by HP-SEC before C-4 analysis. These data, in combination with the HP-SEC data described herein, demonstrate a 1:1 ratio for the EPO-EBP complex.

The complex was obtained as follows. A solution containing 5 mg of EPO and 12 mg of EBP (EBP in excess) was subjected to preparative HP-SEC by the method described above. The first eluting protein peak (EPO-EBP complex) was collected 5 and subjected to analysis by C-4 reversed phase HPLC. Briefly, a 4.6 x 250 mm column (YMC-PACK, C4-AP, 300A) was equilibrated with water/acetonitrile (80%/20%) containing 0.1% trifluoroacetic acid (TFA) and an aliquot of EPO-EBP complex injected at a flow rate of 1 ml/min. After a 5 min wash, a linear gradient of 20-100% acetonitrile containing 0.1% TFA was applied over 20 min and the experiment monitored at 220 nm. Under these conditions, EBP and EPO elute at 14.1 and 15.1 min, respectively, with baseline resolution. A standard curve of each protein was established which demonstrated that detector response was linear over the range of 50 to 500 pmol. The peak area for each protein in the complex was compared to the standard curve to determine the ratio of EPO and EBP in the complex.

### EXAMPLE 5

### Amino Acid Analysis and N-terminal and Peptide Fragment Amino Acid Sequence Analysis.

Amino acid analysis of a solution of purified EBP was performed by standard techniques on an 420H hydrolyzer (Applied Biosystems, Foster City, CA) and the amino acid content estimated using an internal standard. These data were utilized to calculate a molar extinction coefficient of 57,500 for EBP at 280 nm (2.3 absorbance units/mg) based on the absorbance of the protein solution used for amino acid analysis. This value was subsequently used to estimate EBP concentrations. A calculated extinction coefficient (Gill, S.C. and von Hippel, P.H. (1989) Calculation of protein extinction coefficients from amino acid sequence data. Anal. Biochem. 182, 319-326) of 42,760 was originally employed but was subsequently shown to be inaccurate in binding titration experiments.

The amino terminal sequence of EBP was determined on a 2090E Integrated Micro-Sequencing System (Porton Instruments, Fullerton, CA) using standard gas phase Edman degradation chemistry. Repetitive yields were greater than 85%.

For peptide mapping, approximately 50 µg of EBP in PBS was added to 300 µl of 0.05 M TRIS buffer, pH 8.5. To this solution was added 2 µl of 1 mg/ml TPCK-trypsin (Worthington, Freehold, NJ) in water and the mixture was incubated at 37°C for 16 hr. The digestion was quenched with 25 µl trifluoroacetic acid and the mixture filtered through a 0.45 µm membrane filter. The digest was analyzed by HPLC on a Dynamax C-18 5 micron 300A column (Rainin Instrument Co., Woburn, MA) eluted with a gradient of 0-42% acetonitrile/0.1% TFA over 50 min followed by 42-70% of the same buffer over 10 min at 1.0 ml/min. Fractions of material eluting as 215 nm absorbance peaks were collected and the solvent evaporated in a Speedvac. The peptides were sequenced as above.

To determine the binding capacity of the recovered material and confirm the identity and structural integrity of the protein several additional studies were undertaken. These included N-terminal sequence analysis, TOF mass spectrometry and assay formats designed to determine the ligand binding capacity of EBP. N-terminal sequence analysis demonstrated only the expected N-terminal sequence for ten cycles (see above) to confirm the identity of the protein. Sequence analysis of several internal tryptic peptides also confirmed the expected linear sequence of the protein and a principle disulfide bonding pattern of 1-2, 3-4.

### EXAMPLE 6

### Mass Spectrometry.

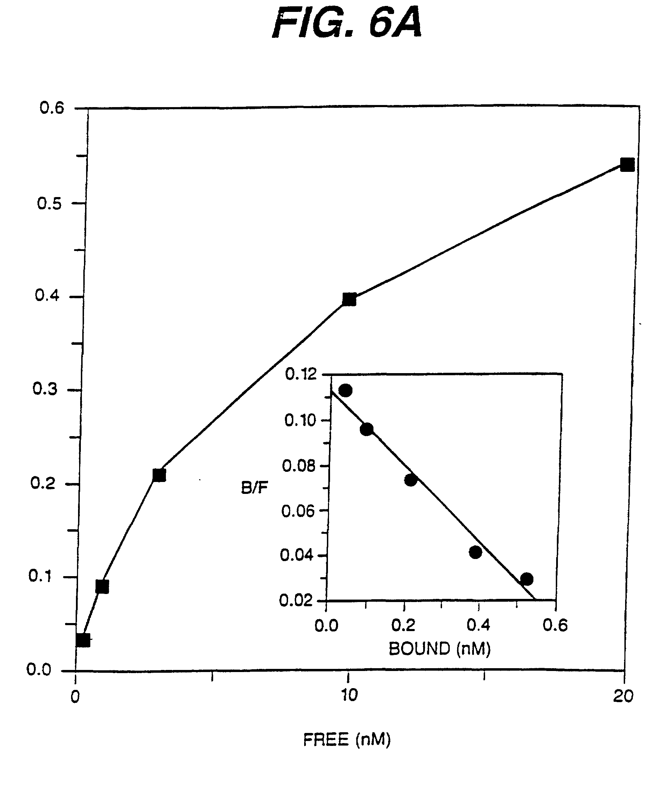

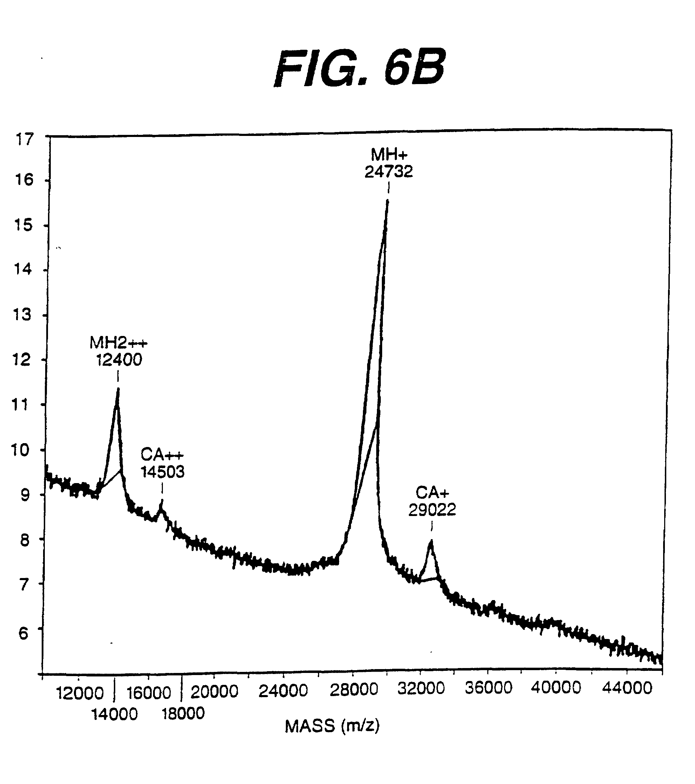

To confirm the mass of the recovered protein, matrix-assisted laser-desorption ionization TOF mass spectrometry was performed on a LaserMAT instrument (Finnigan-MAT, San Jose, CA) utilizing a sinapinic acid matrix and an internal bovine carbonic anhydrase mass standard Figure 6, Panel B demonstrates the MALDI/TOF mass spectrum of EBP showing the molecular ion (MH+) at 24,732 Daltons. The mass centroid of the carbonic anhydrase ion (CA+) was used to calibrate the instrument. Dicharged ions of EBP (MH2++) and carbonic anhydrase(CA++) are also evident in the spectrum. The centroid molecular mass of 24,732 for EBP compares well to the calculated average mass of 24,724 supporting both identity and proper carboxy-cerminal truncation of the protein.

### EXAMPLE 7

### Preparation of EBP Beads and Determination of the Equilibrium Binding Constant (Scatchard Analysis).

EBP as produced by the above method contains one free sulfhydryl group which can be modified without affecting the solution phase binding of ligand. In order to immobilize the EBP for equilibrium binding analysis this observation was extended for the covalent attachment of EBP to agarose beads. The iodoacetyl activation chemistry of Sulfolink beads (Pierce Chemical Co, Rockford, IL) is specific for free thiols and assures that the linkage is not easily reversible. EBP-Sulfolink beads were made as follows: Sulfolink gel suspension (10 ml) was mixed with coupling buffer (40 ml: 50 mM TRIS, pH 8.3, 5 mM EDTA) and the gel was allowed to settle. The supernatant was removed and the EBP (0.3-1 mg/ml in coupling buffer) to be bound was added directly to the washed beads. The mixture was rocked gently for 30 minutes at room temperature, and the beads were allowed to settle for 1 hour at room temperature. The supernatant was removed and saved, and the beads were washed twice with 20 ml of coupling buffer. The washes were retained as well. The beads were then treated with 20 ml of 0.05 M cysteine for 30 minutes at room temperature to block unbound sites. Finally, the beads were washed with 50 ml of 1 M NaCl, then with 30 ml of PBS, and resuspended in 20 ml of PBS and stored at 4°C. The amount of EBP which was covalently bound to the beads was determined by comparing the OD₂₈₀ of the original EBP solution to the total OD₂₈₀ recovered in the reaction supernatant and the two 20 ml washes. Typically, 40-60% of the applied EBP remains associated with the beads.

Binding assays were initiated by the addition of EBP beads (50 µl) to individual reaction tubes. Total binding was measured in tubes containing 0.3-30 nM [¹²⁵I]EPO (NEN Research Products, Boston MA, 100 µCi/µg). For determination of non-specific binding, unlabelled EPO was added at a level of 1000 fold in excess of the corresponding [¹²⁵I]EPO concentration. Each reaction volume was brought to 500 µl with binding buffer (PBS/0.2% BSA). The tubes were incubated for five hours (a time period experimentally determined as adequate for the establishment of equilibrium) at room temperature with gentle rocking. After five hours, each reaction mixture was passed through a 1 ml pipet tip plugged with glass wool. The tubes were washed with 1 ml wash buffer (PBS/ 5% BSA) and this volume as well as two additional 1 ml washes were passed through the pipet tip and collected for determination of the free EPO concentration. Nonspecific binding was determined from the excess cold EPO experiment at each concentration of radiolabel and subtracted from total binding to calculate specific binding. Figure 6, Panel A represents the equilibrium binding data and the inset is the linear transformation (Scatchard) of the data set in Panel A and indicates a Kd of 5 nM ± 2.

### EXAMPLE 8

### Erythropoietin Receptor Competition Binding Analysis.

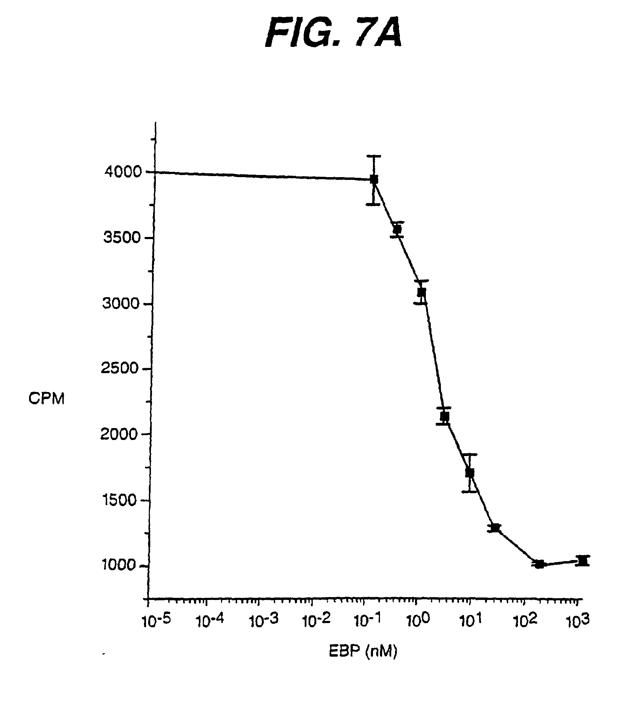

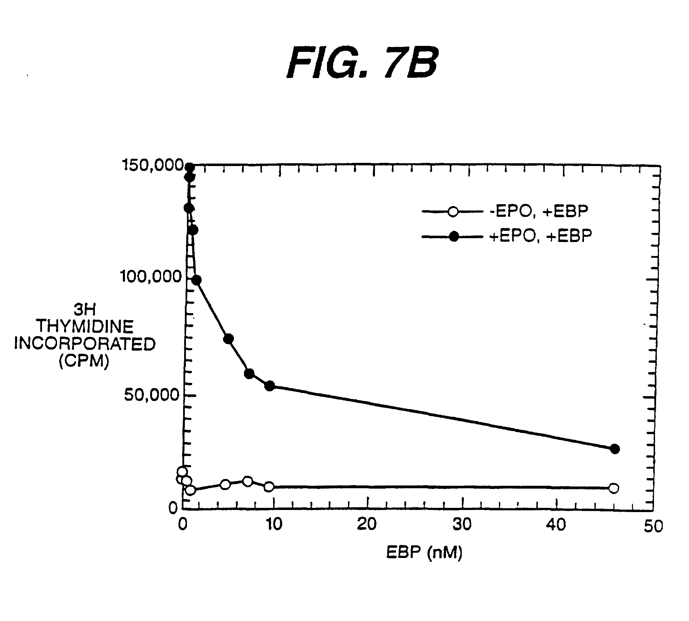

A whole cell-based binding assay was performed by the addition of increasing amounts of purified EBP to a constant amount of [¹²⁵I]EPO and EPOR bearing cells. Nonspecific binding was determined by addition of an excess of EPO (1 µM) and were subtracted prior to data analysis. The estimated IC₅₀ and Kᵢ from five assays were determined to be 1.7 ± 1 nM and 0.94 ± 0.5 nM, respectively. Competition of EBP for [¹²⁵I]EPO binding with intact cell surface EPO receptors yielded an IC₅₀ of ca. 1.7 nM ± 1 (Figure 7, PanelA). Briefly the experiment was performed as follows. TF-1 cells (Kitamura T., Tange T., Teraswa, T., Chiba, S., Kuwaki, T. Miyagawa, K., Piao, Y.-F., Miyazono,K., Urabe, A. and Takaku, F. (1989) Establishment and characterization of a unique human cell line that proliferates dependently on GM-CSF, IL-3 or erythropoietin. J. Cell Physiol. 140, 323-334) were maintained in RPMI 1640, 10% fetal calf serum, 1% L-glutamine, 1% penicillin, 0.1% streptomycin and 1 unit/ml of IL-3 (Genzyme, Cambridge, MA). [¹²⁵I]EPO was obtained from NEN Research Products as described above. Competitive binding studies were performed using a one-stage receptor binding assay method. Briefly, media containing mid-log phase TF-1 cells was centrifuged and the cell pellets resuspended in binding buffer (RPMI 1640, 0.2% BSA, 0.02% sodium azide) at 4 x 10⁷ cells/ml. To assay binding of EPO to the intact cells, with or without competition from EBP, 4 x 10⁶ cells, [¹²⁵I]EPO (typically 80 pM) and EBP were mixed and contained within a final volume of 150 µL, in duplicate. Incubations were conducted in 1.5 ml polypropylene tubes at 10°C overnight. At the end of the incubation period 120 µL aliquots of the binding mixtures were layered onto a 300 µL cushion of 10% sucrose in Sarstedt RIA tubes. Cells were pelleted in a microfuge for 1 min and the tubes were immediately placed in a dry ice bath to quickly freeze the contents. Cells and bound ligand were collected by snipping off the bottom tip of the tube and were transferred to 12 x 75 mm test tubes for quantification of radioactivity in a gamma counter. Lundon-2 (Lundon Software, Chagrin Falls, OH) was employed for data reduction.

### EXAMPLE 9

### Cell Proliferation Neutralization Assay.

Cell line FDC-P1/ER, an EPO-dependent line expressing the murine EPO receptor, was grown and maintained as described previously (Carroll, M.P.,Spivak, J.L., McMahon, M., Weich, N., Rapp, U.R. and May, W.S. 1991. Erythropoietin induces Raf-1 activation and Raf-1 is required for erythropoietin-mediated proliferation. J. Biol. Chem. 266, 14964-14969). The cells were maintained in RPMI 1640 media (Gibco) containing 10% heat-inactivated fetal calf serum and 10 units/ml of recombinant human EPO. For the inhibition of proliferation assay, FDC-P1/ER cells were grown to stationary phase, centrifuged, washed with RPMI 1640 media (no EPO), and plated in media lacking EPO overnight. Assays were set up in 96-well U-bottom tissue culture plates with each assay point in triplicate. Each well contained 4 x 10⁴ cells, 0.5 unit/ml EPO, and various amounts of EBP (in media) in a final volume of 0.2 ml. Plates were incubated at 37°C for ca. 48 hours after which the cells were pulsed with 1 µCi/well of [³P]thymidine (20 Ci/mmol, Dupont-NEN) for 6 hours at 37°C. Cells were harvested onto glass fiber filters using a Tomtec cell harvester. Filters were counted with scintillant using a LKB Betaplate 1205 scintillation counter. The soluble ligand binding domain described herein neutralized the proliferative properties of EPO as detected by the dose dependent neutralization of the proliferative response of EPO in the EPO responsive cell line, FDC-P1/ER. Figure 7, Panel B shows: Inhibition of EPO dependent cellular proliferation by EBP. The IC₅₀ was estimated to be 5 nM ± 1.

Taken together, the examples shown herein, suggest that the refolded EBP produced in the manner described here is highly active and correlates well to the equilibrium binding constant of EBP produced in CHO cells of 1 nM (Yet, M.-G and Jones, S.S. (1993) The Extracytoplasmic Domain of the Erythropoietin Receptor Forms a Monomeric Complex with Erythropoietin. Blood 82, 1713-1719) and the small amount of correctly folded-bacterially expressed EBP-GST fusion protein from an alternative bacterial expression system (Harris, K.W., Mitchell, R.A., and Winkleman, J.C. (1992) Ligand Binding Properties of the Human Erythropoietin Receptor Extracellular Domain Expressed in E. coli. J. Biol.Chem. 267, 15205-15209) but has advantages which include high purity, it is not a fusion protein, it is non-glycosylated, and it is produced and purified in high yield.

### EXAMPLE 10

Peptide Synthesis. Generically, all of the cyclic EPO mimetic peptides described herein were synthesized using Merrifield solid-phase peptide synthesis methodology on Applied Biosytems Models 430A and 431A Peptide Synthesizers. Protected BOC (t-butoxycarbonyl) amino acid derivatives were coupled as hydroxylbenzotriazole esters after acid deprotection (50% Trifluoroacetic acid / 50% dichloromethane) and neutralization (5% diisopropylethylamine /N-methylpyrrolidone) steps. The acid deprotection, neutralization and coupling steps were repeated until the full length peptide-resin was obtained. The completed resin-bound peptide was treated with 50% TFA to yield the free *N*-terminal peptide-resin. The side-chain protecting groups removed and the peptide cleaved from the resin by treatment with liquid HF-anisole (10% by volume) for 90 minutes at -6°C. The free peptide was then precipitated with cold ethyl ether. Additional ether washes were employed to remove residual scavenger (anisole). The dried peptide-resin was then extracted in 50% HOAc, diluted with water and lyophilized. The cysteines were oxidized by suspending the crude peptide 0.1% TFA/acetonitrile followed by dilution with water to a peptide concentration of 1 mg/ml, which yielded a clear solution ( typically 30-50% AcN/water). Solid ammonium bicarbonate was added to the peptide solution until a pH of ca. 8.0 was obtained. Potassium ferricyanide (0.1 M) was added dropwise to the stirred peptide solution until a light yellow solution persisted. This peptide solution was monitored by analytical reverse-phase HPLC for the formation of oxidized product. The cyclic peptides typically eluted after the reduced starting material on a Vydac C18 column. The cyclization reaction time varied from 20 minutes to 18 hours at RT, depending on the particular peptide sequence. Before purification, Eio-Rad AG 2-X8 anion exchange resin (chloride form) was added to the yellow peptide solution to remove the oxidizing agent (iron). The anion exchange resin was removed by filtration and washed with a volume of water equal to the volume of the peptide solution. The combined filtrates were loaded onto a preparative HPLC column (Vydac C18) and the target peptide at 90-95% purity was isolated using a water-acetonitrile gradient with 0.1% TFA. If significant linear peptide was also obtained (typically the reduced peptide eluted closely to the desired cyclic peptide) during chromatography, the oxidation step with potassium ferricyanide was repeated to maximize the overall yield of the reduced target peptide. The fractions collected from the preparative chromatography containing high purity desired peptide were pooled and lyophilized. The final product of each synthesis was characterized by analytical HPLC for purity (usually >95%), ion-spray mass spectroscopy for molecular weight and amino acid analysis for peptide content. All cyclic peptide preparations were negative for free sulfhydryl groups using Ellman's Reagent. The peptides used for the studies disclosed herein are shown in Table 2.

**Table 2**

| EPO mimetic peptides and derivatives | | |
|---|---|---|
| RWJ# | (x) | Peptide sequence* |
| 61233 | | GGTYSCHFGPLTWVCKPQGG [SEQ.ID.NO.:1] |
| 61279 | | YSCHFGPLTWVCK [SEQ.ID.NO.:2] |
| 61177 | | SCHFGPLTWVCK [SEQ.ID.NO.:3] |
| 62021 | 3,5-dibromo-tyr | GGTXSCHFGPLTWVCKPQGG[SEQ.ID.NO.:4] |
| 61718 | | GGLYACHMGPMTWVCQPLRG [SEQ.ID.NQ.:5] |

| | | |
|---|---|---|
| *all peptides are cyclic via an intramolecular disulphide bond and amidated at the -C terminus | | |

Competitive binding assays of peptides were performed as described herein. Individual peptides were dissolved in DMSO to prepare a stock solution of 1 mM. All reaction tubes (in duplicate) contained 50 µL of EBP beads, 0.5 nM [125I]EPO (NEN Research Products, Boston, 100 µCi/µg) and 0-500 µM peptide in a total of 500 µL binding buffer (PBS / 0.2% BSA). The final concentration of DMSO was adjusted to 2.5% in all peptide assay tubes, a value without detectable effect since an examination of the sensitivity of the assay to DMSO demonstrated that concentrations of up to 25% DMSO (V/V) had no deleterious effect on binding. Non-specific binding was measured in each individual assay by inclusion of tubes containing a large excess of unlabelled EPO (1000 nM). Initial assay points with no added peptide were included in each assay to determine total binding. Binding mixtures were incubated overnight at room temperature with gentle rocking. The beads were then collected using Micro-columns (Isolab, Inc.) and washed with 3 mL of wash buffer (PBS / 5% BSA). The columns containing the washed beads were placed in 12 x 75 mm glass tubes and bound radioactivity levels determined in a gamma counter. The amount of bound [¹²⁵I]EPO was expressed as a percentage of the control (total=100%) binding and plotted versus the peptide concentration after correction for non-specific binding. The IC₅₀ was defined as the concentration of the analyte which reduced the binding of [¹²⁵I]EPO to the EBP beads by 50%.

### EXAMPLE 11

EPO Dependent Cell Proliferation Assays. Cell line FDC-P1/hER, an EPO-dependent line expressing the native human EPO receptor was used to determine the EPO mimetic activity of candidate peptides. The cell line exhibits EPO dependent cellular proliferation and the assay was performed as follows. Cells were maintained in RPMI 1640 media (Gibco/BRL) containing 10% heat-inactivated fetal calf serum and 10 units/ml of recombinant human EPO. For the cellular proliferation assay, cells were grown to stationary phase, centrifuged, washed with RPMI 1640 media (no EPO), and plated in EPO minus media for 24 hours.

At 24 hours, the cells were counted, resuspended at 800,000 cells/ml and dispensed at 40,000 cells/well. Stock solutions of the respective peptides (10 mM in DMSO) were prepared and dispensed in triplicate to final concentrations of 1 x 10⁻¹⁰ M through 1 x 10⁻⁵ M and adjusted to a final volume of 0.2 ml. Final DMSO concentrations of 0.1% (V/V, maximal) or less were shown to have no cellular toxicity or stimulatory effects. A standard EPO dose response curve was generated with each assay series. After a 42 hours incubation at 37°C (about 2 cell doublings) 1 uCi/well of [³H] thymidine was added and the incubation continued for 6 hours at which time the cells were harvested and counted to assess [³H]thymidine incorporation as a measure of cell proliferation. Results were expressed as the amount of peptide necessary to yield one half of the maximal activity obtained with recombinant EPO, and are shown in Table 3.

### EXAMPLE 12

Dimerization of EBP by EPO mimetic peptides. Peptide-mediated receptor dimerization was stabilized for study using the non-water soluble homobifunctional sulfhydryl reactive cross linking reagent DPDPB (1,4-di-[2'-pyridyldithio)propionamido]butane, Pierce Chemical Co., Rockford, IL). EBP (22 µM) was incubated in the presence or absence of DPDPB (1.1 mM), 400 µM peptide ligand, 75 µl of PBS, pH 7.5 with all reactions and controls containing a final concentration of 4.4% DMSO and 0.007% TFA. These samples were incubated for 4 hours at room temperature and stored at 4°C for 12 hours before analysis under reducing and non-reducing conditions by SDS-PAGE (10-20% gradient gels, Integrated Separations Systems, Natick, MA).

## Claims

1. A process for purification of erythropoietin binding protein from microbial cell fermentations, comprising:
a) harvesting microbial cells from a fermentation;
b) adding to the harvested microbial cells a sufficient amount of a lysis buffer to produce a crude lysate;
c) collecting insoluble proteins from the crude lysate;
d) solubilizing the insoluble proteins in a solubilization buffer;
e) refolding the solubilized proteins of step d) in a suitable refolding buffer producing a refolded protein;
f) binding the refolded protein of step e) with a hydrophobic interaction chromatography matrix;
g) eluting and collecting active erythropoietin binding protein from the hydrophobic interaction chromatography matrix;
h) contacting the active erythropoietin binding protein from step g) with a size exclusion chromatography matrix;
i) eluting and collecting the active erythropoietin binding protein from the size exclusion chromatography matrix of step h) producing pure erythropoietin binding protein;
j) optionally concentrating and/or diafiltering the product of step i) into a pharmaceutically acceptable carrier; and
k) optionally sterilizing the protein product.

2. The process of claim 1 wherein the lysis solution of step b) is 10mM Tris-HCl, pH7.5, 150mM NaCl, containing 51mg of phenylmethylsulfonyl fluoride, 600mg egg white lysozyme, 1mM MgCl₂ and 12500 units of Benzonase.

3. The process of claim 1 or claim 2 wherein hydrophobic interaction chromatography matrix is phenyl-toyopearl 650 M.

4. The process of any one of claims 1 to 3 wherein the size exclusion chromatography matrix is a high performance size exclusion chromatography matrix, such as Bio-Sil SEC-25 or TosoHaas G3000 SW.

## Patentansprüche

1. Verfahren zur Reinigung von Erythropoietin bindendem Protein aus Mikroben-Zellfermentationen, umfassend:
a) Ernten von mikrobiellen Zellen aus einer Fermentation;
b) Hinzufügen einer ausreichenden Menge eines Lysepuffers zu den geernteten mikrobiellen Zellen, um ein Roh-Lysat herzustellen;
c) Sammeln von unlöslichen Proteinen aus dem Roh-Lysat;
d) Lösen der unlöslichen Proteine in einem Lösungspuffer;
e) Zurückfalten der gelösten Proteine von Schritt d) in einem geeigneten Zurückfaltungspuffer, wobei ein zurückgefaltetes Protein hergestellt wird;
f) Binden des zurückgefalteten Proteins aus Schritt e) mit einer hydrophoben Interaktions-Chromatographie-Matrix;
g) Eluieren und Sammeln von aktivem Erythropoietin bindendem Protein von der hydrophoben Interaktions-Chromatographie-Matrix;
h) in Kontakt bringen des aktiven Erythropoeitin bindenden Proteins aus Schritt g) mit einer Größenausschluß Chromatographie-Matrix;
i) Eluieren und Sammeln des aktiven Erythropoietin bindenden Proteins von der Größenausschluß-Chromatographie-Matrix von Schritt h), wobei ein reines Erythropoietin bindendes Protein hergestellt wird;
j) gegebenenfalls, Konzentrieren und/oder Diafiltrieren des Produkts von Schritt i) in einem pharmazeutisch akzeptablen Träger; und
k) gegebenenfalls, Sterilisieren des Proteinprodukts.

2. Verfahren nach Anspruch 1, wobei die Lyselösung von Schritt b) 10mM Tris-HCl, pH 7,5, 150mM NaCl, enthaltend 51 mg von Phenylmethylsulfonylfluorid, 600 mg Eiweiß-Lysozym, 1mM MgCl₂ und 12500 Einheiten Benzonase® ist.

3. Verfahren nach Anspruch 1 oder Anspruch 2, wobei die hydrophobe Interaktions-Chromatographie-Matrix Phenyl-Toyoperl 650 M ist.

4. Verfahren nach einem der Ansprüche 1-3, wobei die Größenausschluß-Chroinatographie-Matrix eine High-Performance Größenausschluß-Chromatographie-Matrix wie z.B. Bio-Sil SEC-25 oder TosoHaas G3000 SW ist.

## Revendications

1. Procédé de purification de la protéine liant l'érythropoïétine, à partir de fermentations de cellules microbiennes, comprenant :
a) la récolte des cellules microbiennes d'une fermentation ;
b) l'addition aux cellules microbiennes récoltées d'une quantité suffisante d'un tampon de lyse pour produire un lysat brut ;
c) la collecte des protéines insolubles du lysat brut ;
d) la solubilisation des protéines insolubles dans un tampon de solubilisation ;
e) le repliage des protéines solubilisées de l'étape d) dans un tampon approprié de repliage produisant une protéine repliée ;
f) la liaison de la protéine repliée de l'étape e) avec une matrice de chromatographie par interaction hydrophobe ;
g) l'élution et la collecte de la protéine liant l'érythropoiétine active de la matrice de chromatographie par interaction hydrophobe ;
h) la mise en contact de la protéine liant l'érythropoïétine active de l'étape g) avec une matrice de chromatographie par exclusion de taille ;
i) l'élution et la collecte de la protéine liant l'érythropoïétine active de la matrice de chromatographie par exclusion de taille de l'étape h) pour produire la protéine pure de liaison de l'érythropoïétine ;
j) facultativement, la concentration et/ou la diafiltration du produit de l'étape i) dans un support pharmaceutiquement acceptable ; et
k) facultativement, la stérilisation de la protéine produite.

2. Procédé de la revendication 1, où la solution de lyse de l'étape b) est Tris-HCl 10 mM, pH 7,5, NaCl 150 mM contenant 51mg de fluorure de phénylméthylsulfonyle, 600 mg de lysozyme de blanc d'oeuf, MgCl₂ 1 mM et 12500 unités de Benzonase.

3. Procédé de la revendication 1 ou de la revendication 2, où la matrice de chromatographie par interaction hydrophobe est phényl-toyopearl 650 M.

4. Procédé de l'une quelconque des revendications 1 à 3, où la matrice de chromatographie par exclusion de taille est une matrice de chromatographie par exclusion de taille haute performance comme Bio-Sil SEC-25 ou TosoHaas G3000 SW.
